# EUROPEAN PATENT APPLICATION

(11) **EP 0 730 033 A2**
(43) Date of publication of application: **04.09.1996**
(21) Application number: 96102907.1
(22) Date of filing: 27.02.1996
(51) Int. Cl.: C12P 19/18, C12P 19/14

(54) **Method of producing glycosphingolipid**

(30) Priority: 03.03.1995 JP 70762/95
(71) Applicant: SNOW BRAND MILK PRODUCTS CO., LTD., Sapporo-shi, Hokkaido 065 (JP)
(72) Inventor: Suguri, Toshiaki, Tokyo (JP); Yanahira, Shuichi, Tsurugashima-shi, Saitama (JP); Hanagata, Goro, Sayama-shi, Saitama (JP); Tatsumi, Kiyoshi, Iruma-shi, Saitama (JP)
(74) Representative: Boeters, Hans Dietrich, Dr.

(57) **Abstract**

A method of producing a glycosphingolipid by reacting glycosidase with oligosaccharide comprising two or more saccharide or a glycoside and ceramide as substrates and transferring saccharide residue to ceramide through transglycosylation. By simple operations, a highly pure glycosphingolipid can be obtained with little byproduct.

## Description

### Background of the Invention

### Field of the Invention

The present invention relates to a method of producing glycosphingolipid from oligosaccharide comprising two or more saccharides and ceramide or glycoside and ceramide by using glycosidase.

### Description of the Prior Art

Glycolipid is a generic name of a substance comprising saccharide and lipid as composing ingredients, has not only a hydrophilic portion but also a hydrophobic portion in a single molecule and exists on a cell surface etc. It is known that roles of glycolipid in a living body relate to intercellular recognition, cell division, cell proliferation, cell differentiation and immunity. In addition, they are thought to play an important role with respect to physiological function. In order to investigate on the above field, synthesis of glycolipid having a naturally occurring sugar structure or novel glycolipid has been tried.

Though a method of producing glycosphingolipids such as lactosylceramide by chemical synthesis is well known so far, these methods require many processes, complexed operations, and have high risk by using toxic substances and produce many by-products.

Alternatively, there is a method of using glucosyltransferase or galactosyltransferase which is an intracellular enzyme transferring sugar to ceramide. However,these enzymes are present only in a small amount in a living body, generally unstable, purified by complex processes comprising several steps of column chromatography and difficult to prepare in a large scale. Moreover, in an enzymic reaction, a sugar nucleotide such as uridine diphosphate (hereinafter abbreviated as UDP)-glucose or UDP-galactose is necessary as a sugar donor substrate and is expensive. In addition, because of high substrate specificity of glycosyltransferase, it is possible to prepare a glycosphingolipid having a naturally occurring structure but not a novel glycosphingolipid which has not a naturally occurring structure.

In the case of preparing glycosphingolipids from natural sources, it is very difficult to separate and purify an individual glycosphingolipid because the content of glycosphingolipids in natural sources is generally low and a variety of sphingolipids are present therein.

On the other hand, an enzyme called by glycosidase which hydrolyzes a glycosidic bond of a carbohydrate is known to add a sugar to an acceptor through a transglycosylation reaction and used for synthesis of oligosaccharide. And in Japanese published unexamined patent application 99494 (1992) are disclosed a method of producing glycoshingoplipid from oligosaccharide and ceramide by a condensation reaction using an enzyme which acts on glycosphingolipid to cleave a glycoside bond between sugar and ceramide, releasing oligosaccharide itself (the enzyme can be available commercially by the name of endoglycoceramide or ceramideglycanase). It is not tried yet to produce a glycosphingolipid by transferring sugar to a ceramide molecule through transglycosylation by glycosidase which has a releasing action of monosaccharide from a carbohydrate.

### Summary of the Invention

The present invention is accomplished by eager investigation on a novel method of producing a glycosphingolipid. That is, the present inventors found a novel method of producing glycosphingoglipid by acting glycosidase on oligosaccharide comprising two or more saccharides or glycoside and ceramide and bind sugar residue with ceramide through transglycosylation by glycosidase and accomplished the invention. Accordingly, an object of the present invention is to provide a simple and safe method of producing a glycosphingolipid with little by-product from an oligosaccharide or glycoside and ceramide through transglycosylation by glycosidase.

The method of producing a glycosphingolipid of the present invention is characterized by acting glycosidase on a substrate such as oligosaccharide comprising two or more saccharides or glycoside and ceramide and binding sugar residue with ceramide through transglycosylation by glycosidase.
The reaction formula of the tansglycosylation of the present invention is described as follows:

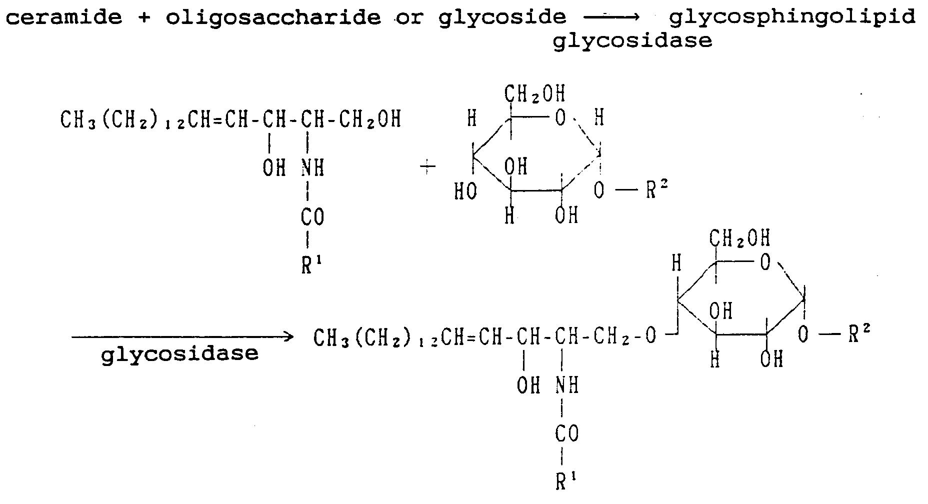

Wherein R¹-CO represents a fatty acid residue and R² represents a saccharide(s) residue.

### Detailed Description of Preferred Embodiments

Various kinds of glycosidases can be used as enzyme source of the present invention. For example, β-galactosidase and β-glucosidase etc. can be exemplified. The origin of β-glycosidase is not restricted specifically, but β-galactosidase derived from Bacillus circulans or β-glucosidase derived from ahmond can be preferably used.

As for a ceramide as a substrate of the present invention, glycosphingolipid-degraded substance or sphingomyelin degraded substance derived from bovine brain or chemically synthesized one can be exemplified.

As for a sugar donor comprising a larger oligosaccharide or glycoside than disaccharide used as a substrate of the present invention, a suitable oligosaccharide can be used depending on the species of glycosidase. For example, when β-galactosidase is used as a glycosidase, lactose, para-nitrophenyl galactoside or ortho-nitrophenyl galactoside etc. can be exemplified. In the case of using β-glucosidase as a glycosidase, salicin, cellobiose, para-nitrophenyl glucoside, ortho-phenylglucoside can be exemplified as a substrate.

The method of producing glycosphingolipid of the present invention will be described in detail below. To a solution comprising more than 0.5%, preferably more than 10 % of sugar donor such as oligosaccharide comprising two or more saccharides or glycoside which is one of the substrates, a ceramide solution dissolved by using a surface activating agent such as sodium taurocholate, sodium taurodeoxycholate or sodium cholate was added to make a substrate solution. An appropriate glycosidase is added to the substrate solution to add a sugar residue to ceramide by reacting for 10 min.-10 days. The optimum pH and optimum temperature for the reaction has to be considered depending on a glycosidase species used. Normally, a reaction is carried out at pH 3-8 and at 5-90°C. Glycosidase with titer of 0.1 mU/mg - 1,000,000 U/mg can be used. Thus, often the reaction is carried out for 10 min.-10 days, glycosphingolipid can be produced in the reaction mixture. In addition, by using an appropriate buffer solution as a substrate solution used in a normal enzymic reaction, various kinds of sugar residue can be added to ceramide.

For example, by using lactose as a sugar donor and β-galactosidase as a glycosidase, galactosyl ceramide as a glycosphinoglipid can be produced. Alternatively, by using cellobiose as a sugar donor and β-glucosidase as a glycosidase, glycosylceramide as a glyocosphingolipid can be produced. These glycosphingolipids produced like the above can be identified by thin layer chromatography. The sphingoglycolipid produced like the above can be purified by removing taurodeoxycholate, enzyme, saccharides and buffer solution from the reaction mixture through an ODS column chromatography, followed by purification by silica-gel chromatography. A highly pure glycosphingolipid whose purity is more than 90 % can be obtained by the above procedures.

The glycosphingolipid obtained by the method of the present invention can be used only by removing sugar donor, ceramide, enzyme, buffer solution, surface activating agent etc. as a glycosphingolipid as it is or after condensation or drying, because the reaction mixture contains little by-product and the content of glycosphingolipid therein is high.
By the method of the present invention, a glycosphingolipid can be prepared by an enzymic reaction in which transglycosylation by glycosidase is utilized with a larger oligosaccharide and glycoside than disaccharide as a substrate. Then, a glycosphingolipid can be prepared by comparatively simple operations. Moreover, there is little by-product and a highly pure glycosphingolipid can be easily obtained from the reaction mixture. And purification thereof can be carried out easily, so that it is possible to supply cheap glycosphingolipid as a medicinal source or a reagent.

### Examples

The present invention will be described in detail by showing examples as follows:

### [Example 1]

Three hundreds milligrams of lactose and 10 mg of ceramide (derived from bovine brain) were suspended in 700 µl of 0.1 M acetate buffer solution(pH 5.0) comprising 2%(w/v) taurodeoxycholic acid. To this suspension, 15 U of β-galactosidase derived from Bacillus ciculans( Daiwa-kasei co.,Ltd.) was added and kept at 50°C for 24 hours. After the reaction was over, lactose, saccharides degraded by the enzymic reaction, taurodeoxycholic acid, β-galactosidase and buffer were removed from the reaction mixture through an Octadecylsilane(hereinafter referred to ODS) (sep-pack-C18) column chromatography and, further, ceramide etc. were removed by silica gel column chromatography to produce purified galactosylceramide. It was confirmed by thin layer chromatography that a glycosphingolipid having the same mobility as that of galactosylceramide, that is, a glycosphingolipid derived from bovine brain was produced and purity thereof was high(higher than 95%).

### [Example 2]

Two hundreds mg of cellobiose and 10 mg of ceramide (derived from bovine brain) were suspended in 800 µl of 0.1M acetate buffer solution (pH 5.0) comprising 2 %(w/v) taurodeoxycholic acid. To this solution, 15 U β-glucosidase derived from ahmond (Sigma) was added and kept for 24 hours. After the reaction was over, cellobiose, saccharides degraded in the enzymic reaction, taurodeoxycholic acid, β-glucosidase and buffer solution were removed from the reaction mixture by ODS (sep-pack C18) column chromatography and ,further,ceramide was removed by silicagel chromatography to produce purified glucosylceramide. It was confirmed by thin layer chromatography that a glycosphingolipid having the same mobility as that of glycosylceramide, that is, glycosphingolipid derived from milk was produced and purity thereof was high (higher than 95%).

## Claims

1. A method of producing a glycosphingolipid characterized by reacting glycosidase with oligosaccharide comprising two or more saccharides or glycoside and ceramide as a substrate, transferring sugar residue to ceramide through transglycosylation to yield a glycosphingolipid.

2. The method according to claim 1 wherein said transglycosylation is carried out at pH 3-8 and at 5-90°C.

3. The method according to claim 1 wherein the titer of said glycosidase used is 0.1mU/mg - 1,000,000 U/mg.

4. The method according to claim 1 wherein said glycosidase is β-galactosidase.

5. The method according to claim 1 wherein said glycosidase is β-galactosidase derived from Bacillus ciraculans.

6. The method according to claim 1 wherein said glycosidase is β-galactosidase derived from ahmond.

7. A method of producing galactosylceramide characterized by reacting β-galactosidase with lactose and ceramide as substrates, transferring galactose residue to ceramide through transglycosylation to yield galactosylceramide.

8. A method of producing glucosylceramide characterized by reacting β-glucosidase with cellobiose and ceramide as substrates, transferring glucose residue to ceramide through transglycosylation to yield glucosylceramide.

9. A method of producing a glycosphingolipid characterized by reacting glycosidase with oligosaccharide comprising two or more saccharides or glycoside and ceramide as substrates, transferring saccharide residue to ceramide through transglycosylation to yield a sphingoglycolipid, passing the reaction mixture through a ODS (octadecylsilane) column, following by silica gel chromatography, collecting the eluent thereof and obtaining highly pure sphingoglycolipid.
